(19) Europäisches Patentamt
European Patent Office
Office européen des brevets

(11) **EP 3 181 760 B1**

(12) **EUROPEAN PATENT SPECIFICATION**

(45) Date of publication and mention
of the grant of the patent:
**11.10.2023 Bulletin 2023/41**

(21) Application number: **15887496.6**

(22) Date of filing: **27.03.2015**

(51) International Patent Classification (IPC):
**C02F 1/30** *(2023.01)*  **C02F 103/02** *(2006.01)*
**C02F 103/08** *(2006.01)*  **E02D 31/06** *(2006.01)*
**B08B 17/02** *(2006.01)*  **B63B 59/04** *(2006.01)*
**E02B 17/00** *(2006.01)*

(52) Cooperative Patent Classification (CPC):
**C02F 1/30; E02D 31/06;** B63B 59/04;
C02F 2103/023; C02F 2103/08; C02F 2303/20;
E02B 17/0017; Y02A 20/144

(86) International application number:
**PCT/JP2015/059803**

(87) International publication number:
**WO 2016/157343 (06.10.2016 Gazette 2016/40)**

(54) **METHOD FOR PREVENTING FORMATION OF BIOFILM**

VERFAHREN ZUR VERHINDERUNG DER BILDUNG VON BIOFILM

PROCÉDÉ EMPÊCHANT LA FORMATION DE BIOFILM

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**21.06.2017 Bulletin 2017/25**

(73) Proprietors:
• **The Chugoku Electric Power Co., Inc.**
**Hiroshima-shi, Hiroshima 730-8701 (JP)**
• **Sessile Research Corporation**
**Himeji-shi, Hyogo 672-8023 (JP)**

(72) Inventors:
• **YANAGAWA, Toshiharu**
**Hiroshima-shi**
**Hiroshima 730-8701 (JP)**
• **SAITO, Shinsuke**
**Himeji-shi**
**Hyogo 672-8023 (JP)**
• **YAMASHITA, Keiji**
**Himeji-shi**
**Hyogo 672-8023 (JP)**
• **KAMIYA, Kyoko**
**Himeji-shi**
**Hyogo 672-8023 (JP)**
• **HAYASHI, Yoshio**
**Himeji-shi**
**Hyogo 672-8023 (JP)**

(74) Representative: **Gill Jennings & Every LLP**
**The Broadgate Tower**
**20 Primrose Street**
**London EC2A 2ES (GB)**

(56) References cited:
EP-A1- 3 180 978       WO-A1-2007/107722
WO-A1-2014/027402      WO-A1-2014/188347
JP-A- 2009 172 051     JP-A- 2009 507 525
JP-A- 2010 207 278     JP-A- 2012 228 627
JP-U- 3 111 579

EP 3 181 760 B1

**Description**

Technical field

The present invention relates to methods of suppressing bio-film formation on a structure in water.

Background art

In power plants, such as thermal or nuclear power plants that use seawater as a coolant, bio-films are easily formed in such a place as heat exchangers of condenser tubings. An increased amount of formed bio-films may possibly cause a problem such as reduction in heat exchange efficiency of the heat exchangers.
Accordingly, a chlorine-based substance such as a sodium hypochlorite solution or chlorine dioxide is conventionally injected into the coolant (see, for example, Non-patent literatures 1 to 3).

Related art documents

Non-patent literature

Non-patent literature 1: "Kaisei Seibutsu Oson Taisaku Manual (Manual to control fouling by marine organisms)," Marine Biofouling Control Committee, The Electrochemical Society of Japan ed., Gihodo Shuppan Co., Ltd, 1991.
Non-patent literature 2: "Hatsudensho Kaisui Setsubi no Oson Taisaku Handbook (Handbook to control fouling in or on seawater equipment of power plants," Thermal and Nuclear Power Engineering Society ed., Kouseisha-kouseikaku Corporation, 2014.
Non-patent literature 3: Atsushi KAWABE, "Ogata fuchaku seibutsu taisaku gijyutu souran (Comprehensive list of techniques to control large sessile organisms)," Marine Biofouling Control Committee, The Electrochemical Society of Japan, 1998.

Further prior art of relevance to the subject-matter of the appended claims is known from WO 2014/188347 A1, which discloses a method of anti-fouling of a protected surface using light irradiation with wavelength between 220 and 420 nm, and from WO 2014/027402 A1, which discloses a method of stopping a larva of Pteriomorphia in a settlement stage from swimming or crawling in seawater using irradiating light in a wavelength range including 400 to 550 nm.

Summary of the invention

Problems to be solved by the invention

An object of the present invention is to provide methods of suppressing bio-film formation on a structure in water.

Means to solve the problems

One aspect of the present invention is a method of suppressing formation of a bio-film on a structure in water including irradiating light comprising the spectrum of 409 to 412 nm to the structure. The light can comprise the spectrum of a part of 400 to 440 nm. The light has a peak wavelength in the range between 409 nm and 412 nm.
The light has a spectrum with a spectral irradiance at the structure of 1.4 $\mu Wcm^{-2}nm^{-1}$ or higher in the range between 409 nm and 412 nm. The light can comprise the spectrum of 400 to 420 nm. It is preferable that the irradiance of the light is 3 $Wm^{-2}$ or higher. It is preferable that the light is not a laser beam. The light may be an LED beam. The water is seawater.

Brief description of the drawings

Fig. 1 is a graph showing a relationship between irradiance and photon flux density measured in the air, in an Example of the present invention;
Fig. 2 is a graph showing a spectral irradiance at each substrate plate in an Example of the present invention;
Fig. 3 is a graph showing the attenuation of irradiance during the propagation of LED light through seawater in an Example of the present invention;

Fig. 4 shows photographs of a net made of CREMONA® yarns attached to a substrate plate in an Example of the present invention;

Fig. 5A shows photographs of bio-films formed on substrate plates 1 week after the substrate plates were placed in seawater in order to support the illustration of an Example of the present invention;

Fig. 5B shows photographs of bio-films formed on substrate plates 2 weeks after the substrate plates were placed in seawater in order to support the illustration of an Example of the present invention;

Fig. 5C shows photographs of bio-films formed on substrate plates 3 weeks after the substrate plates were placed in seawater in order to support the illustration of an Example of the present invention;

Fig. 5D shows photographs of bio-films formed on substrate plates 16 weeks after the substrate plates were placed in seawater in order to support the illustration of an Example of the present invention;

Fig. 6 is a graph showing the number of bacteria counted, using DAPI staining, on glass slides placed between adjacent substrate plates and collected in 5 to 7 days in an Example of the present invention;

Fig. 7 is a graph showing a relationship between the density of bacteria attached to glass slides placed between adjacent substrate plates and collected in 5 to 7 days and the distance from the center of an area irradiated with LED light in an Example of the present invention;

Fig. 8 is a graph showing the coverage measured, using an illuminator, on glass slides placed between substrate plates, collected in 5 to 7 days, and stained with crystal violet; and

Fig. 9 is a graph showing the coverage measured, using a stereomicroscope, on glass slides placed between substrate plates, collected in 5 to 7 days, and stained with crystal violet.

Embodiment for carrying out the invention

The objects, features, advantages, and ideas of the present invention are apparent to those skilled in the art from the description of the present specification. Furthermore, those skilled in the art can easily reproduce the present invention from the description of the present specification. The mode and the specific example described below represent a preferable embodiment of the present invention, which is given for the purpose of illustration or description. The present invention is not limited thereto. It is obvious to those skilled in the art that various changes and modifications may be made according to the descriptions of the present specification without departing from the scope of the present invention defined by the appended claims.

Methods of suppressing bio-film formation on a structure in water according to the present invention include the step of irradiating light comprising the spectrum of 409 to 412 nm to the structure.

As used herein, the structure is not specifically limited as long as it is located in water, and examples include water intake systems, rotary screens, bar screens, drum screens, shellfish filtration systems, mussel filters, net screens, water intake pumps, circulating water pumps, circulating water pipes, heat exchangers, condensers, bearing cooling water coolers, lubricating oil coolers, LNG vaporizers, power generators, sponge ball cleaning units, water discharge systems, seawater temperature gauges, residual chlorine meters, water quality meters, jellyfish protection nets, waterwheels, impellers, valves, rotation shafts, water intake channels, filtration tanks, and membranes of thermal power plants, nuclear power plants, tidal power plants, wave power plants, ocean current power plants, ocean thermal energy conversion plants, hydroelectric power plants, seawater pumped hydroelectric energy storage plants, LNG plants, oil refinery plants, or seawater desalination plants; dams; ships; hulls, screws, ballast tanks, ballast water intake and discharge pipes, and pumps in shipyards; water tanks, pipes, pumps, sand filter tanks, net bait wells, ropes, and seaweed farming nets in aquaculture facilities, experimental aquaculture facilities, fisheries facilities, aquariums, or fish and shellfish farming tanks; buoys; floating piers; floats; fixed fishing nets; and water installations of washing machines, bathrooms, and washrooms in houses, water treatment facilities, hot springs, public bathes, and hydroponics factories. Water itself and surfaces of farmed organisms such as fishes, oysters, and scallops are also examples of the structure.

The term bio-film refers to an organization formed by a microorganism on a surface of a structure in water. Bio-films are typically formed like a film and include extracellular polymeric substances (EPSs) such as polysaccharides secreted by microorganisms. The bio-films may include dead bodies, excreta, feces and tubes of microorganisms. Examples of microorganisms include organisms belonging to bacteria, fungi, cyanobacteria, and protozoa. Furthermore, microalgae such as attached diatoms, green algae, brown algae, and red algae as well as larvae of sponges, hydroids, jellyfishes, tube-dwelling bristle worms, moss animals, and gammaridean amphipods are also included in microorganisms.

The light to be irradiated to a structure in water comprises the full spectrum of 409 to 412 nm. It can comprise the spectrum of a part of 400 to 440 nm. It should be noted that the term "a part" is intended not to include "full". It is preferable that the light comprise the full spectrum of 400 to 412 nm and may comprise the spectrum of ultraviolet radiation (having wavelengths shorter than 400 nm), visible radiation (having wavelengths of 400 to 830 nm), or infrared radiation (having wavelengths longer than 830 nm). Light with wavelengths in the range between 400 nm and 420 nm can be transmitted better through seawater than ultraviolet light. Accordingly, the present invention can exert effects of light on a larger area compared with methods using light in the ultraviolet range only. In addition, as shown in Examples, the light has a peak wavelength in the range between 409 nm and 412 nm. This light may not be necessarily a laser beam.

The irradiance of the light is not specifically limited and can appropriately and easily be determined by those skilled in the art depending on the environment to be irradiated (e.g., quality, depth, and clarity of the water). It is preferable to irradiate light with an irradiance of 3 $Wm^{-2}$ or higher, and light with an irradiance of about 200 $Wm^{-2}$ or higher is more preferable. The light with a spectral irradiance of about 1.4 $\mu Wcm^{-2}nm^{-1}$ or higher at the sessile organisms in the range between 409 nm and 412 nm is used in the method of the invention, and the light with a spectral irradiance of about 500 $\mu Wcm^{-2}nm^{-1}$ or higher is more preferable. The light irradiation time is also not specifically limited and can appropriately and easily be determined by those skilled in the art depending on the environment to be irradiated. The irradiation time can be set at, for example, 3 seconds or longer, 10 seconds or longer, 30 seconds or longer, 100 seconds or longer, or 5 minutes or longer. The irradiation can be continuous or intermittent. When the irradiation is intermittent, the total light irradiation time is preferably as described above.

The irradiation method is not particularly limited, but devices such as an LED emitter, a mercury lamp, and a fluorescent tube can be used as the irradiator. An LED emitter is preferred and optical fibers using LEDs are particularly preferable.

Examples

<1> Preparation of model channel

In this Example, a model channel of 7 m long, 10 cm wide, and 10 cm deep was disposed in a place on an offshore facility of Hiroshima Prefectural Fisheries and Marine Technology Center, and seawater was run through the channel at a flow rate of 0.1 m/sec. An LED light unit (with SMD LEDs manufactured by CCS Inc.; LED panel model name: ISL-150X150-VV-TPHI/BTFN; light-emitting surface: 15 cm × 15 cm; the number of SMDs: 120 (i.e., 8 by 15); the size of SMD: 5 × 5 mm; peak wavelength: 409 to 412 nm; viewing angle characteristics: half-power angle of 58.6°, half width of 14 nm) was disposed on a sidewall of the channel and light was emitted horizontally into the channel through a diffuser and a silica glass (of 5 mm thick). A light shaping diffuser (Optical Solutions Corp., LSD60×1PC10-F5, 1.25 mm thick, substrate; polycarbonate) with which a beam is shaped in an elliptical pattern (diffusion angle 60° × 1°) was mounted between the panel and the silica glass. The LED light was diffused using this light shaping diffuser and irradiated into the channel. The LED light was irradiated through an opening of 10 cm × 10 cm (square) formed in the sidewall of the channel.

Next, substrate plates (each having a width of 10 cm and a height of 15 cm) were placed on the other sidewall of the channel. First, a substrate plate (E) was placed right in front of the LED panel. Four substrate plates were placed on either side of E every 25 cm (with a gap of 15 cm between the adjacent substrate plates) (i.e., A to D and F to I on the opposite sides of E from the upstream to the downstream), and additional four plates (J to M) were placed on the downstream side every 100 cm (with a gap of 90 cm between the adjacent substrate plates) to eventually line up the substrate plates in the order of A to M from the upstream to the downstream.

During the experiments, light from outside to the channel was blocked, and the LED light was adjusted so that the irradiance was about 200 $Wm^{-2}$ at the center of the substrate plate E (base point) facing opposite to the LED panel. Measurements of the light at the start of the experiment (March 6, 2014, 15:00) are given in Table 1. As a control, a channel within which light from outside was blocked and no LED light was irradiated was used (with substrate plates A' to M').

[Table 1]

| Substrate plate | Photon flux density | Irradiance | Illuminance | Peak wavelength | Peak spectral irradiance |
|---|---|---|---|---|---|
| | $\mu mols^{-1}m^{-2}$ | $Wm^{-2}$ | lx | nm | $\mu Wcm^{-2}nm^{-1}$ |
| A | 0.24 | 0.079 | 0.32 | 407 | 0.011 |
| B | 0.83 | 0.146 | 0.30 | 407 | 0.035 |
| C | 3.62 | 0.460 | 2.55 | 407 | 0.160 |
| D | 27.19 | 3.121 | 20.58 | 407 | 1.430 |
| E | 1768.5 | 199.687 | 970 | 406 | 506.238 |
| F | 27.13 | 3.114 | 20.29 | 407 | 1.394 |

(continued)

| Substrate plate | Photon flux density | Irradiance | Illuminance | Peak wavelength | Peak spectral irradiance |
|:---:|:---:|:---:|:---:|:---:|:---:|
| | $\mu mols^{-1}m^{-2}$ | $Wm^{-2}$ | lx | nm | $\mu Wcm^{-2}nm^{-1}$ |
| G | 3.54 | 0.451 | 2.23 | 407 | 0.155 |
| H | 0.84 | 0.147 | 0.57 | 407 | 0.031 |
| I | 0.24 | 0.079 | 0.48 | 407 | 0.011 |
| J | 0.02 | 0.054 | 0.21 | 406 | 0.001 |
| K | 0.00 | - | 0.027 | - | - |
| L | 0.00 | - | 0.00 | - | - |
| M | 0.00 | - | 0.00 | - | - |
| * J to M: 0.4 $\geq$ | | | | | |

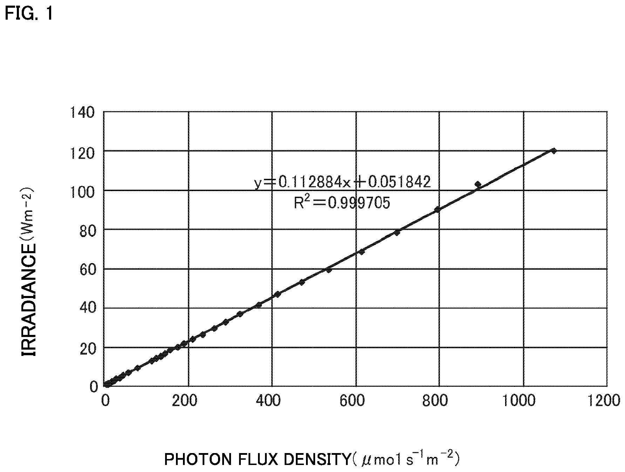

The irradiance in seawater was calculated by measuring a photon flux density in seawater and then using the following equation (I) obtained from a relationship between the irradiance and the photon flux density measured in the air in advance (Fig. 1).

$$\text{Irradiance } (\overline{W}m^{-2}) = 0.112884 \times \text{photon flux density } (\mu mols^{-1}cm^{-2})$$
$$+ 0.051842 \ldots \text{(I)}$$

During the experimental period, the irradiance at the substrate plate E was adjusted to 200 $Wm^{-2}$ every observation day by setting the photon flux density in seawater at 1768.5 $\mu mols^{-1}cm^{-2}$.

The following devices were used for the measurements.

(1) Photon flux density: Underwater quantum sensor LI-192SA (400 to 700 nm) manufactured by MEIWAFOSIS Co., Ltd.
(2) Spectral irradiance: Multi-purpose spectroradiometer MSR-7000N (200 to 2500 nm) manufactured by Opto Research Corporation
(3) Illuminance: Illuminance meter T-10WL (spectral wavelength range of 400 to 700 nm) manufactured by Konica Minolta Sensing Inc.

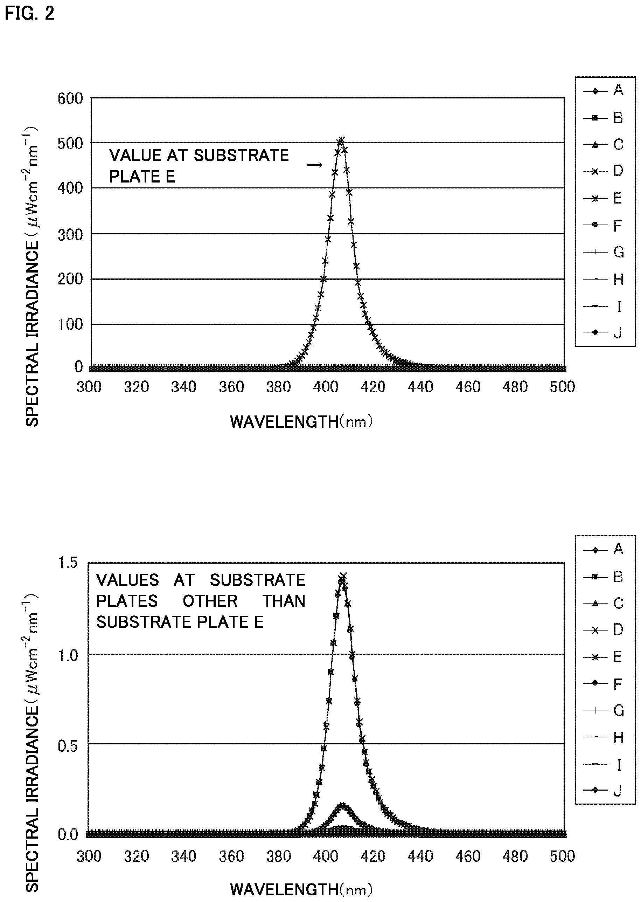

A relationship between the wavelength of the LED light and the spectral irradiance at the substrate plates is shown in Fig. 2. The peak was within the wavelength range of 409 to 412 nm at all substrate plates.

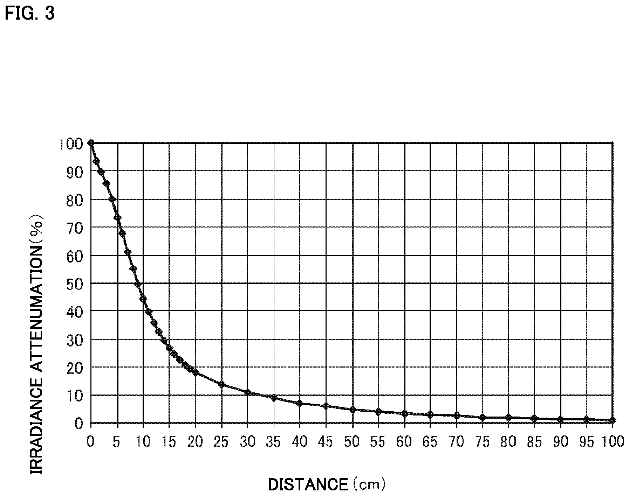

The irradiance of the LED light in seawater shown in Table 1 was attenuated to 73.2% at a position 5 cm away from the panel and to 44.43% at a position 10 cm away from the panel (Fig. 3). Accordingly, when the irradiance at the substrate plate E is 200 $Wm^{-2}$, the irradiance at a position 0 cm away from the panel is 450 $Wm^{-2}$ and the irradiance at the center of the channel (5 cm away) is 325 $Wm^{-2}$.

Illumination of the LED light actually reached a 30-cm-wide range over the substrate plates facing to the LED panel. Since the seawater was run at a flow rate of 0.1 m/sec., larvae in the seawater pass through the illuminated area over 1-3 seconds, during which they are irradiated with light with the irradiance of 200 to 450 $Wm^{-2}$ for at least 1 second.

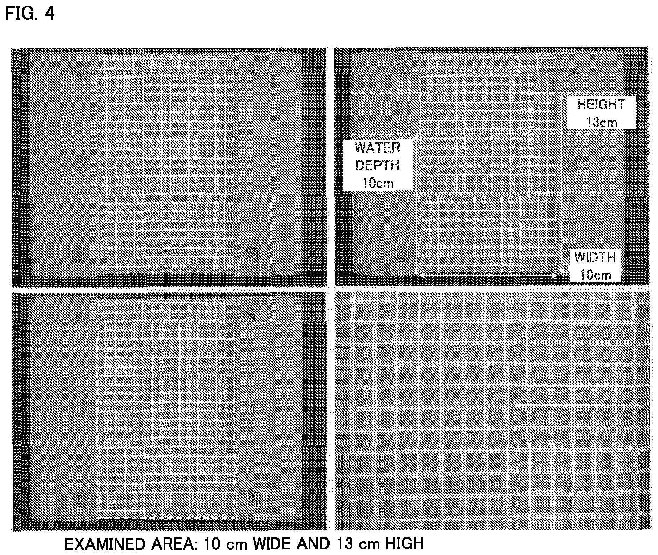

A net made of CREMONA® yarns (knotless net manufactured by NITTO SEIMO Co., Ltd. ; yarn diameter of 1 mm, and mesh size of 5 mm) was attached to each substrate plate (Fig. 4). Each substrate plate is 10 cm wide and immersed 10 cm into water within the channel. The sessile organisms, however, settled up to 13 cm height, above the surface of the seawater. Accordingly, the 13-cm span from the bottom of the water is subjected to examination, that is, the substrate plate is examined on the area of 130 $cm^2$. This examined area has 16 warps and 20 wefts. The number of mesh squares (5 mm $\times$ 5 mm) is 300 (15 squares in column and 20 squares in row) per single examined area.

<2> Suppression of bio-film formation by irradiation of LED light

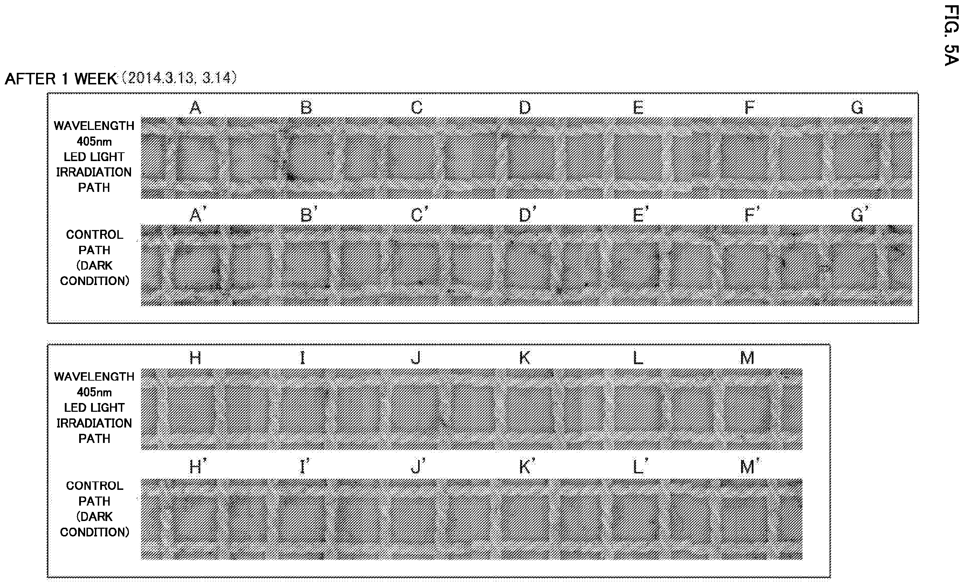

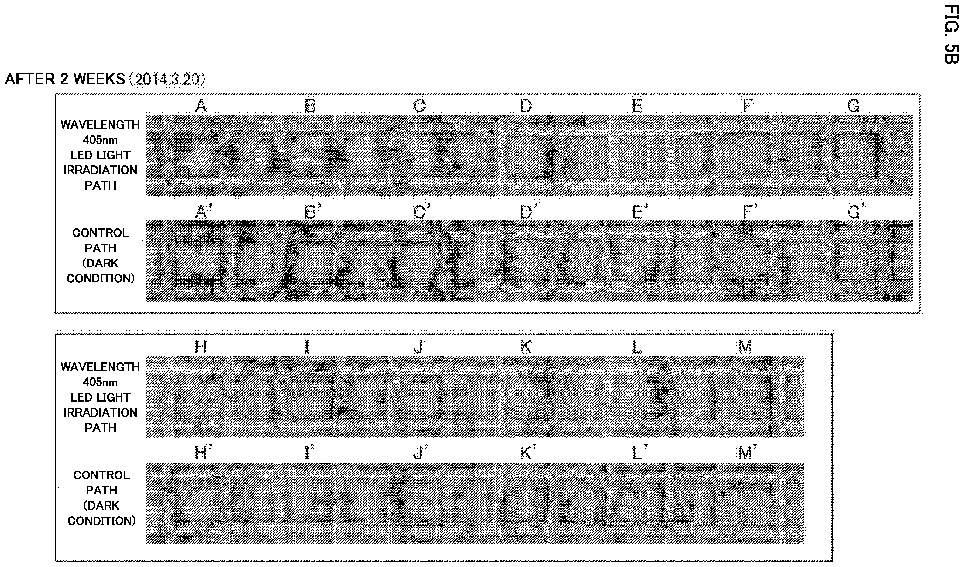

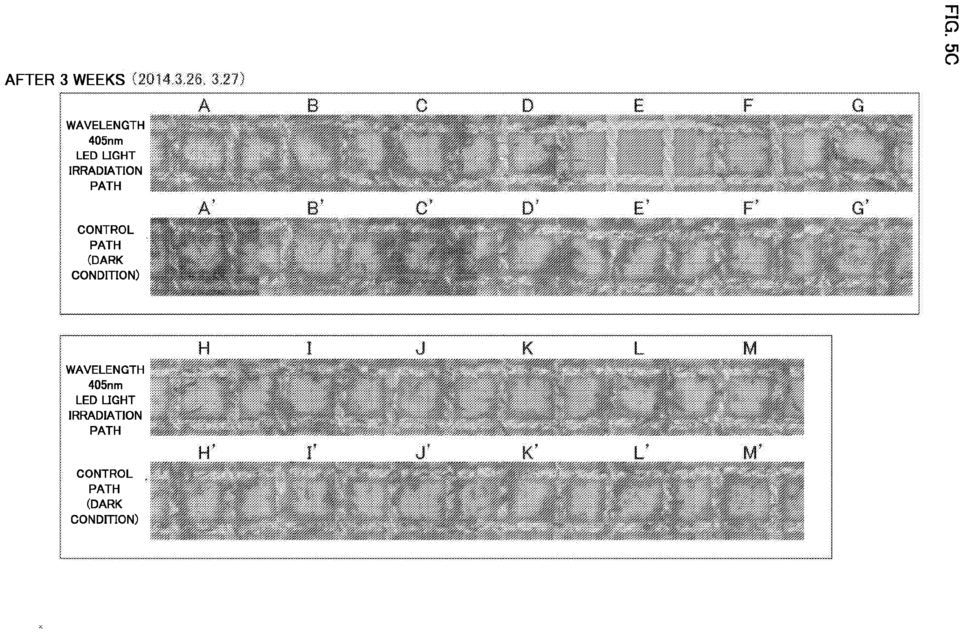

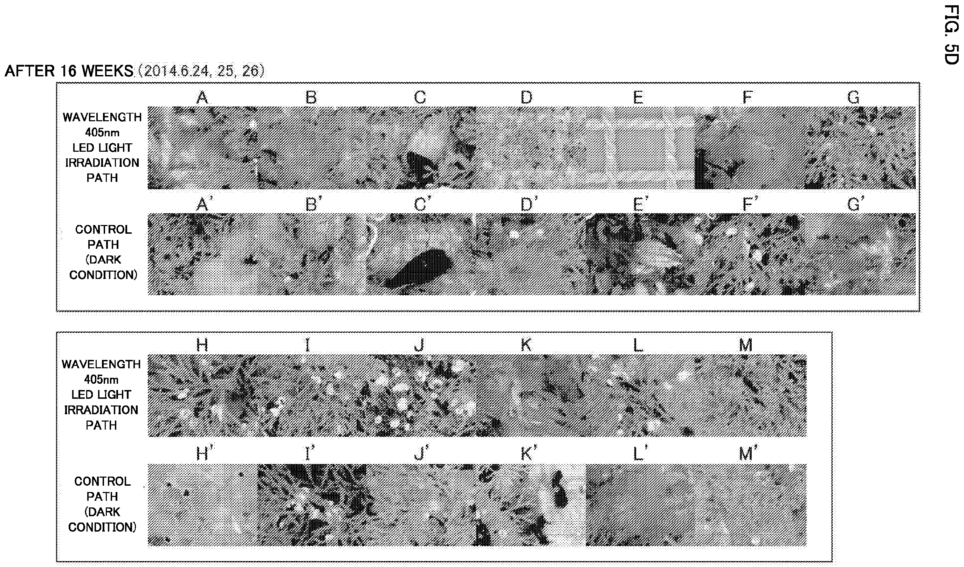

Bio-films formed on the substrate plates thus arranged were observed 1, 2, and 16 weeks later. Appearances of the substrate plates at those times are shown in Fig. 5. Bio-film formation was suppressed on the substrate plate (E) as apparent from the photographs taken after 2 weeks. No bio-film was formed on the substrate plate (E) even after 16 weeks. Suppression of bio-film formation was found on the substrate plates (D) and (F), although being less than that on (E).

During this period, glass slides (76 mm × 26 mm) were vertically (with their longitudinal axis aligned with the vertical direction) placed at the center in a gap between the adjacent substrate plates. The following experiments were performed on the glass slides collected after 5 to 7 days. The glass slides were designated using the alphabetical indications of the substrate plates on both sides of the glass slide (for example, a glass slide is designated by AB when it is placed between the substrate plates A and B).

(1) Counting the number of bacteria by DAPI (4',6-diamidino-2-phenylindole) staining

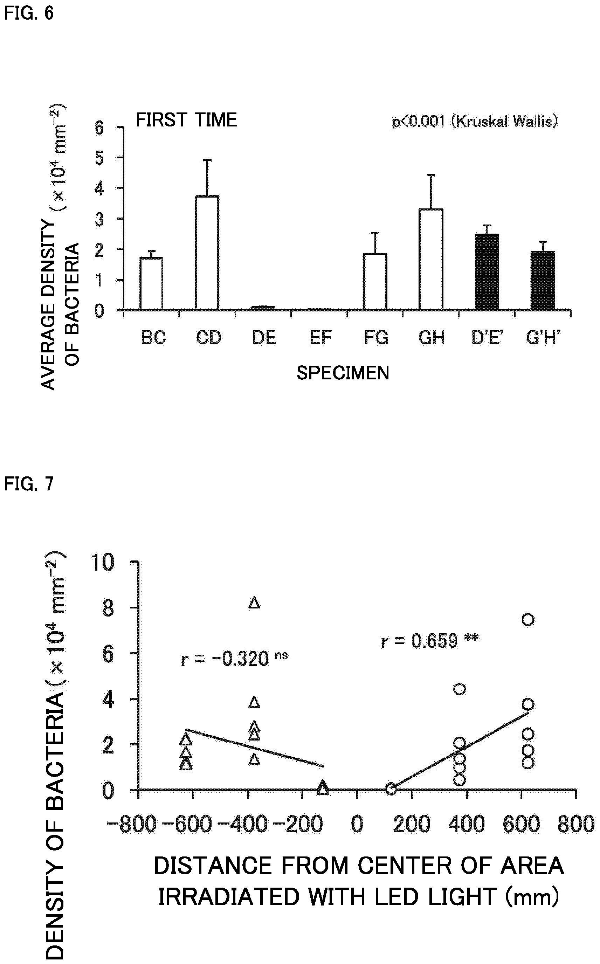

The glass slides stored in a dry condition were immersed in TBS for 5 minutes. Then, 200 pl of 0.05 mg/ml DAPI staining solution was added dropwise thereto and reacted in a dark place for 10 minutes. After the staining, excessive DAPI staining solution was washed out of the glass slide with TBS, and a cover glass was placed. Photographs were taken using a reflected light microscope camera systems (Olympus BX51 & C3040Z) (oil immersion objective with 100x magnification, WU excitation). Photographs of central portions of the glass slides were taken 5 times by moving them sufficiently in one direction so as not to overlap the photographed areas. A stage micrometer was also photographed to calculate an image pickup area (i.e., 0.12 mm × 0.09 mm). The images thus taken were visually observed on a screen of a personal computer to count the number of bacteria. As a result, as shown in Fig. 6, the glass slides at the positions DE and EF had significantly small numbers of bacteria.

Fig. 7 shows a relationship between the density of the bacteria attached to the glass slides and the distance from the center of the area irradiated with an LED beam. The correlation coefficient on the negative side was not significant, but the density of the attached bacteria tended to increase as the photographed area is far from the light source in the upstream and downstream directions. (2) Staining with crystal violet

Glass slides were immersed in a 0.5% crystal violet solution containing 2% EtOH for 1 minute, washed with purified water for 10 minutes and then dried.

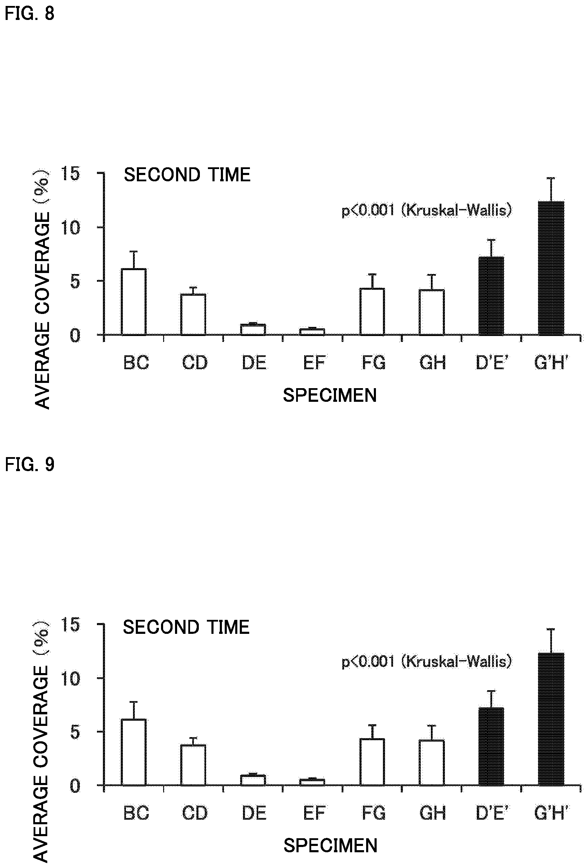

The glass slides stained with crystal violet were placed on an illuminator (HAKUBA KLV-7000 or SINKOHSHA SV540A) and photographs of the overall surface were taken using a digital camera (Olympus BX51). Using a photograph editor software product (Photoshop 5.0 LE, Adobe), an area of 40 mm × 10 mm fixed at the center was cut out from each photographed image and used as an examined area. The examined area was binarized and the number of pixels in a dark area (bio-film) was counted using an image analyzer software product (ImageJ 1.48v, National Institutes of Health) to calculate coverage (%). Examples of the result are shown in Fig. 8, in which DE and EF always exhibited low values.

(3) Stereomicroscope imaging

Glass slides stained with crystal violet as in the experiment method 2 were photographed with a transmitted light using a stereomicroscope imaging system (Olympus SZX & C3040Z). At that time, glass slides were placed on window-opened plates that were randomly arranged and the center of each window was photographed. Objective micrometers were photographed as well, and the photographed area was calculated (1.35 mm × 1.01 mm). Then, the images were analyzed as performed in (2). Examples of the results are shown in Fig. 9, in which DE and EF always exhibited low values as in (2).

Thus, although with some differences in the extent, suppression of bio-film formation was observed on the substrate plates (D) to (F). Bio-film formation could be completely suppressed in the cases with a stronger effect.

Industrial applicability

The present invention made it possible to provide methods of suppressing bio-film formation on a structure in seawater.

**Claims**

1. A method of suppressing bio-film formation on a structure in seawater, comprising irradiating light comprising the spectrum of 409 to 412 nm to the structure, wherein the light has a spectral irradiance at the structure of 1.4 $\mu Wcm^{-2}nm^{-1}$ or higher and a peak wavelength in the range between 409 nm and 412 nm.

2. The method according to Claim 1, wherein the light comprises the spectrum of a part of 400 to 440 nm.

3. The method according to Claims 1 or 2, wherein the irradiance of the light is 3 Wm$^{-2}$ or higher.

4. The method according to any one of the Claims 1 to 3, wherein the light is irradiated from an LED emitter, a mercury lamp, or a fluorescent tube.

**Patentansprüche**

1. Verfahren zum Unterdrücken der Bildung von Biofilm auf einer Struktur in Meerwasser, umfassend Bestrahlung der Struktur mit Licht, umfassend das Spektrum von 409 bis 412 nm, wobei das Licht eine spektrale Bestrahlungsstärke an der Struktur von 1,4 $\mu$Wcm$^{-2}$nm$^{-1}$ oder höher und eine Spitzenwellenlänge in dem Bereich zwischen 409 nm und 412 nm aufweist.

2. Verfahren nach Anspruch 1, wobei das Licht das Spektrum eines Teils von 400 bis 440 nm umfasst.

3. Verfahren nach Anspruch 1 oder 2, wobei die Bestrahlungsstärke des Lichts 3 Wm$^{-2}$ oder höher ist.

4. Verfahren nach einem der Ansprüche 1 bis 3, wobei das Licht von einem LED-Strahler, einer Quecksilberlampe oder einer Leuchtstoffröhre abgestrahlt wird.

**Revendications**

1. Procédé de suppression de la formation de biofilm sur une structure dans l'eau de mer, comprenant l'irradiation d'une lumière comprenant le spectre de 409 à 412 nm sur la structure, dans lequel la lumière présente une irradiance spectrale au niveau de la structure supérieure ou égale à 1,4 $\mu$Wcm$^{-2}$nm$^{-1}$ et une longueur d'onde maximale comprise entre 409 nm et 412 nm.

2. Procédé selon la revendication 1, dans lequel la lumière comprend le spectre d'une partie de 400 à 440 nm.

3. Procédé selon la revendication 1 ou 2, dans lequel l'irradiance de la lumière est supérieure ou égale à 3 Wm$^{-2}$.

4. Procédé selon l'une quelconque des revendications 1 à 3, dans lequel la lumière est irradiée à partir d'un émetteur LED, d'une lampe à mercure ou d'un tube fluorescent.

FIG. 1

PHOTON FLUX DENSITY($\mu$mol s$^{-1}$m$^{-2}$)

## FIG. 2

FIG. 3

FIG. 4

EXAMINED AREA: 10 cm WIDE AND 13 cm HIGH

FIG. 5A

EP 3 181 760 B1

AFTER 1 WEEK (2014.3.13, 3.14)

|  | A | B | C | D | E | F | G |
|---|---|---|---|---|---|---|---|
| WAVELENGTH 405nm LED LIGHT IRRADIATION PATH | | | | | | | |

|  | A' | B' | C' | D' | E' | F' | G' |
|---|---|---|---|---|---|---|---|
| CONTROL PATH (DARK CONDITION) | | | | | | | |

|  | H | I | J | K | L | M |
|---|---|---|---|---|---|---|
| WAVELENGTH 405nm LED LIGHT IRRADIATION PATH | | | | | | |

|  | H' | I' | J' | K' | L' | M' |
|---|---|---|---|---|---|---|
| CONTROL PATH (DARK CONDITION) | | | | | | |

FIG. 5B

EP 3 181 760 B1

AFTER 2 WEEKS (2014.3.20)

| | A | B | C | D | E | F | G |
|---|---|---|---|---|---|---|---|
| WAVELENGTH 405nm LED LIGHT IRRADIATION PATH | | | | | | | |

| | A' | B' | C' | D' | E' | F' | G' |
|---|---|---|---|---|---|---|---|
| CONTROL PATH (DARK CONDITION) | | | | | | | |

| | H | I | J | K | L | M |
|---|---|---|---|---|---|---|
| WAVELENGTH 405nm LED LIGHT IRRADIATION PATH | | | | | | |

| | H' | I' | J' | K' | L' | M' |
|---|---|---|---|---|---|---|
| CONTROL PATH (DARK CONDITION) | | | | | | |

FIG. 5C

EP 3 181 760 B1

AFTER 3 WEEKS (2014.3.26, 3.27)

FIG. 5D

AFTER 16 WEEKS (2014.6.24, 25, 26)

WAVELENGTH 405nm LED LIGHT IRRADIATION PATH

CONTROL PATH (DARK CONDITION)

A B C D E F G
A' B' C' D' E' F' G'

WAVELENGTH 405nm LED LIGHT IRRADIATION PATH

CONTROL PATH (DARK CONDITION)

H I J K L M
H' I' J' K' L' M'

FIG. 6

FIG. 7

FIG. 8

FIG. 9

## REFERENCES CITED IN THE DESCRIPTION

**Patent documents cited in the description**

- WO 2014188347 A1

- WO 2014027402 A1

**Non-patent literature cited in the description**

- Kaisei Seibutsu Oson Taisaku Manual (Manual to control fouling by marine organisms). Gihodo Shuppan Co., Ltd, 1991
- Hatsudensho Kaisui Setsubi no Oson Taisaku Handbook (Handbook to control fouling in or on seawater equipment of power plants. Kouseisha-kouseikaku Corporation, 2014

- **ATSUSHI KAWABE.** Ogata fuchaku seibutsu taisaku gijyutu souran (Comprehensive list of techniques to control large sessile organisms). Marine Biofouling Control Committee, The Electrochemical Society of Japan, 1998